# EUROPEAN PATENT SPECIFICATION

(11) **EP 2 081 884 B1**
(45) Date of publication and mention of the grant of the patent: **22.05.2013**
(21) Application number: 07818474.4
(22) Date of filing: 26.09.2007
(51) Int. Cl.: C07C 209/36, C07C 211/61

(54) **PROCESS FOR THE PREPARATION OF AMINES**
VERFAHREN ZUR HERSTELLUNG VON AMINEN
PROCEDE POUR LA PREPARATION D'AMINES

(30) Priority: 28.09.2006 CH 15452006
(43) Date of publication of application: 29.07.2009
(73) Proprietor: Syngenta Participations AG, 4058 Basel (CH)
(72) Inventor: WALTER, Harald, 4332 Stein (CH); TOBLER, Hans, 4054 Basel (CH); GIORDANO, Fanny, 4333 Muenchwilen (CH); ZELLER, Martin, 4333 Muenchwilen (CH)
(74) Representative: Thwaite, Jonathan Simon
(86) International application number: PCT/EP2007/008391
(87) International publication number: WO 2008/037460

(56) References cited:
- WO-A-2006/037632

## Description

The present invention relates to a process for the preparation of syn-enriched 5-amino-9-isopropyl-benzonorbornene.

The syn-enriched amine 5-amino-9-isopropyl-benzonorbornene is an important intermediate that can be used in the preparation of syn-enriched 3-difluoromethyl-1-methyl-1 H-pyrazole-4-carboxylic acid (9-isopropyl-benzonorbornen-5-yl)amide. That syn-enriched amide is a fungicide which is described in WO 04/35589 and WO 06/37632.

WO 06/37632 describes a process for the preparation of syn-enriched 5-amino-9-isopropyl-benzonorbornene that uses the unsaturated nitro compound isopropylidene-5-nitro-benzonorbornadiene as a direct precursor (see Scheme 1):

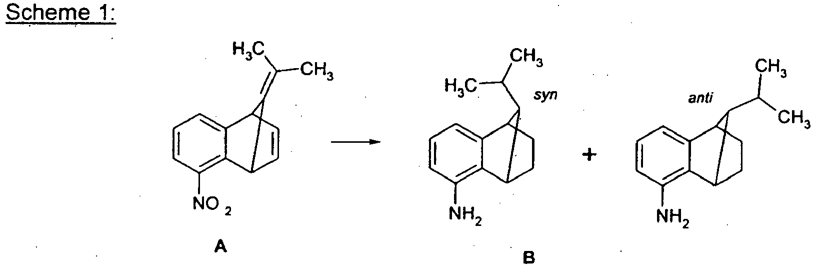

According to WO 06/37632, isopropylidene-5-nitro-benzonorbornadiene (A), the preparation of which is described in the same document, can be reduced to the desired amine (B) catalytically under standard conditions. Suitable catalysts that are mentioned for the reduction are Rh/C, Rh/Al₂O₃, Rh₂O₃, Pt/C, PtO₂, Pd/C, Ir(COD)Py(Pcy) and Ra/Ni. According to WO 06/37632, the choice of catalyst is decisive for the syn/anti ratio of the resulting amine. The catalysts Rh/C, Rh/Al₂O₃, Rh₂O₃, Pt/C and PtO₂ result in syn-enriched amines. For example, a syn/anti ratio of 9:1 is obtained using Rh/C as catalyst and tetrahydrofuran as solvent (see Examples H3 and H4, page 41 of WO 06/37632). According to the teaching of WO 06/37632, the catalysts Pd/C, Ir(COD)Py(Pcy) and Ra/Ni result in anti-enriched amines. Thus, with Pd/C as catalyst and using the same solvent (tetrahydrofuran), a syn/anti ratio of 3:7 is obtained at normal pressure.

In view of the high cost of rhodium and platinum catalysts, a process that uses those catalysts is unsuitable for the production of syn-enriched 5-amino-9-isopropyl-benzonorbornene, especially for the large-scale preparation of fungicides.

The aim of the present invention is accordingly to provide a process for the preparation of syn-enriched 5-amino-9-isopropyl-benzonorbornene that makes it possible to prepare that compound in a high yield and good quality in a cost-effective way.

Surprisingly, it has been found that by the selection of a particular pressure range in conjunction with the selection of a particular temperature range, palladium catalysts then have syn-steering properties.

The present invention therefore relates to the provision of a process for the preparation of syn-enriched 5-amino-9-isopropyl-benzonorbornene in which the ratio of compounds of formula la (syn) 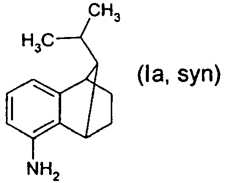 to compounds of formula Ib (anti) 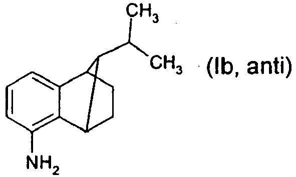 is from 70:30 to 99:1, which process comprises reacting a compound of formula II 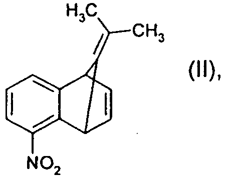 in a pressure vessel with hydrogen in the presence of a palladium catalyst and a solvent, at a pressure of at least 2 bar and a temperature of from 0°C to 45°C, to form syn-enriched 5-amino-9-isopropyl-benzonorbornene.

5-Amino-9-isopropyl-benzonorbornene is a chiral molecule and can occur in four stereoisomeric forms. Those forms are the enantiomers of formulae I_{I}, I_{II}, I_{III}, and I_{IV}:

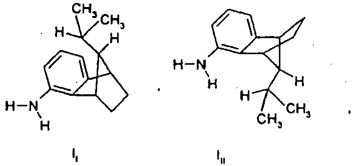

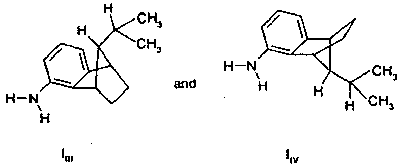

In the context of the present invention, compounds of formula la (syn) 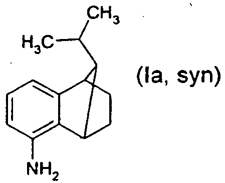 are understood to be compounds of formula I_{I}; compounds of formula I_{II}; or a mixture, in any ratio, of compounds of formula I_{I} and compounds of formula I_{II}. In the context of the present invention, compounds of formula la (syn) are understood to be, preferably, a racemic mixture of compounds of formula I_{I} and compounds of formula I_{II}.

In the context of the present invention, compounds of formula Ib (anti) 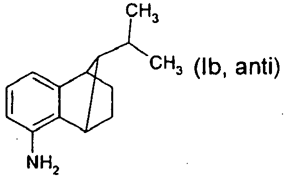 are understood to be compounds of formula I_{III}; compounds of formula I_{IV}; or a mixture, in any ratio, of compounds of formula I_{III} and compounds of formula I_{IV}. In the context of the present invention, compounds of formula Ib (anti) are understood to be, preferably, a racemic mixture of compounds of formula I_{III} and compounds of formula I_{IV}.

In the context of the present invention, a "racemic mixture" of two enantiomers is understood to be a mixture of the two enantiomers in a ratio substantially equal to 1 : 1.

The process according to the invention is suitable especially for the preparation of syn-enriched 5-amino-9-isopropyl-benzonorbornene in which the ratio of compounds of formula la (syn) to compounds of formula Ib (anti) is from 80:20 to 99:1, especially preferably from 85:15 to 99:1.

In a further embodiment, the process according to the invention is suitable especially for the preparation of syn-enriched 5-amino-9-isopropyl-benzonorbornene in which the ratio of compounds of formula la (syn) to compounds of formula Ib (anti) is from 70:30 to 90:10, especially preferably from 80:20 to 90:10, and very especially from 85:15 to 90:10.

The reaction according to the invention is carried out in a pressure vessel, such as an autoclave.

For example, hydrogen is fed into the reaction mixture in which the palladium catalyst or its precursor is already present until the desired pressure is reached; moreover, the desired pressure is maintained for the whole of the reaction time. For that purpose, hydrogen is fed in and the hydrogen consumption is monitored for the whole of the reaction time. In that embodiment, the reaction is terminated when there is no further hydrogen absorption. The compounds of formula I have then been exhaustively hydrogenated under the chosen reaction conditions.

In a further embodiment, the reaction is terminated once a desired amount of hydrogen has been consumed, more especially 5 equivalents based on the compound of formula II.

The palladium catalysts used may be heterogeneous or homogeneous palladium catalysts.

A suitable heterogeneous palladium catalyst is, for example, a palladium catalyst supported on a carrier. The carrier is inert under the conditions of the reaction according to the invention. Suitable carriers are, for example, carbon, aluminium oxide, silicon oxide, barium carbonate, barium sulfate and calcium carbonate. Suitable heterogeneous palladium catalysts may have various water contents; they may have a water content of up to 80 % (w/w).

Homogeneous palladium catalysts are typically produced *in situ.* Suitable precursors of homogeneous palladium catalysts are, for example, palladium acetate, palladium chloride, palladium hydroxide and palladium oxide.

Suitable amounts of palladium catalyst for this reaction are, for example, from 0.01 to 10 mol% based on the compound of formula II, especially from 0.1 to 1 mol%.

In the context of the present invention "solvent" is understood to be an inert compound that is fluid under the conditions of the reaction according to the invention. Preferred solvents are alcohols, ethers, esters, hydrocarbons and mixtures thereof. The solvents may optionally be halogenated.

Preferred alcohols are methanol, ethanol, propanol, isopropanol and glycol; methanol is especially preferred. Preferred ethers are tetrahydrofuran, dioxane and anisole, especially tetrahydrofuran. Preferred esters are ethyl acetate, methyl acetate, butyl acetate and dimethyl carbonate. Preferred hydrocarbons are benzene, xylene and toluene. Examples of solvent mixtures are methanol/tetrahydrofuran mixtures and methanol/ethanol mixtures.

Preferred halogenated solvents are trifluoroethanol and chlorobenzene.

Preferred solvents are alcohols, ethers and mixtures thereof.

In one embodiment of the present invention, the reaction is carried out in the presence of from 0.01 to 10 equivalents of an additive, preferably from 0.2 to 3 equivalents. "Equivalents" relates to the compound of formula II. Suitable additives are bases or acids.

Suitable bases are organic bases, such as, for example, organic nitrogen bases. Suitable organic nitrogen bases are, for example, trialkylamine bases, such as triethylamine, trimethylamine, Hünig's base, N-methylpyrrolidine, N-methylmorpholine and N-methylpiperidine.

Suitable acids are strong inorganic acids, for example mineral acids such as hydrochloric acid or sulfuric acid, or strong organic acids, for example organic sulfonic or carboxylic acids, such as methanesulfonic acid or trifluoroacetic acid.

In one embodiment of the present invention, trialkylamine bases, such as, for example, triethylamine, are used as additives.

The reaction according to the invention is carried out at a pressure of at least 2 bar. Preference is given to a pressure of from 2 to 50 bar, special preference to a pressure of from 5 to 50 bar, and very special preference to a pressure of from 7 to 50 bar.

In one embodiment of the reaction according to the invention, a pressure of from 7 to 20 bar, preferably from 7 to 15 bar, is used; very especially preferred in that embodiment is a pressure of from 8 to 12 bar.

The temperatures of the reaction according to the invention are in a range from 0°C to 45°C, with preference being given to a range from 20°C to 45°C and special preference to a range from 20°C to 30°C.

The reaction time of the reaction according to the invention is generally from 1 to 100 hours.

The reaction according to the invention can be carried out with or without the isolation of intermediates. If non-exhaustively hydrogenated intermediates are isolated after a first reaction step, the reaction conditions of a subsequent second step, such as, for example, the solvent, can be varied. Preferably, the reaction according to the invention is carried out without isolation of intermediates.

The starting compounds of formula II are known and can be prepared as described, for example, in WO 06/037632.

The present invention is explained in greater detail by way of the following Examples:

### Example P1: Preparation of syn-enriched 5-amino-9-isopropyl-benzonorbornene at ambient temperature and a pressure of 10 bar:

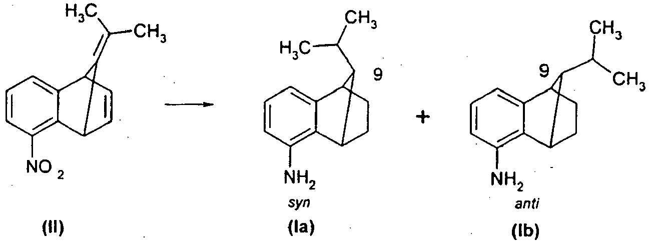

In an autoclave, 500 mg (2.2 mmol) of isopropylidene-5-nitro-benzonorbomadiene in 5 ml of solvent are exhaustively hydrogenated in the presence of 5% Pd/C (15 mg) at ambient temperature and 10 bar for 18 h with magnetic stirring. A mixture of the syn/anti-5-amino-9-isopropyl-benzonorbornenes is obtained. The identity of the reaction product, its diastereoisomeric purity (in % syn) and yield (in %) are ascertained by means of gas chromatography.

| **Solvent** | **% syn** | **Yield** |
|---|---|---|
| Methanol | 83 | 98 |
| Ethanol | 81 | 98 |
| Tetrahydrofuran | 84 | 91 |
| Dioxane | 76 | 58 |
| Anisole | 80 | 87 |
| Ethyl acetate | 86 | 90 |
| Methyl acetate | 83 | 59 |
| Dimethyl carbonate | 85 | 85 |

### Example P2: Preparation of syn-enriched 5-amino-9-isopropyl-benzonorbornene at ambient temperature and a pressure of 20 bar:

In an autoclave, 500 mg (2.2 mmol) of isopropylidene-5-nitro-benzonorbomadiene in 5 ml of solvent are exhaustively hydrogenated in the presence of 5% Pd/C (25 mg) at ambient temperature and 20 bar for 18 h with magnetic stirring. A mixture of the syn/anti-5-amino-9-isopropyl-benzonorbornenes is obtained. The identity of the reaction product, its diastereoisomeric purity (in % syn) and yield (in %) are ascertained by means of gas chromatography.

| **Solvent** | **% syn** | **Yield** |
|---|---|---|
| Toluene | 78 | 94 |
| Trifluoroethanol | 77 | 94 |

### Example P3: Yield in the preparation of syn-enriched 5-amino-9-isopropyl-benzonorbornene as a function of the reaction pressure:

In an autoclave, 500 mg (2.2 mmol) of isopropylidene-5-nitro-benzonorbomadiene in 5 ml of methanol are exhaustively hydrogenated in the presence of 5% Pd/C (15 mg or 25 mg) at ambient temperature for 18 h with magnetic stirring. A mixture of the syn/anti-5-amino-9-isopropyl-benzonorbornenes is obtained. The identity of the reaction product, its diastereoisomeric purity (in % syn) and yield (in %) are ascertained by means of gas chromatography.

| **Reaction pressure** | **Amount of Pd/C** | **% syn** | **Yield** |
|---|---|---|---|
| 1.5 bar | 25 mg, 5% w/w | 63 | 90 |
| 5 bar | 25 mg, 5% w/w | 78 | 97 |
| 10 bar | 15 mg, 3% w/w | 83 | 98 |
| 20 bar | 15 mg, 3% w/w | 86 | 98 |
| 40 bar | 25 mg, 5% w/w | 84 | 100 |
| 60 bar | 25 mg, 5% w/w | 86 | 100 |

### Example P4: Yield in the preparation of syn-enriched 5-amino-9-isopropyl-benzonorbornene as a function of reaction temperature:

In an autoclave, 500 mg (2.2 mmol) of isopropylidene-5-nitro-benzonorbomadiene in 5 ml of methanol are exhaustively hydrogenated in the presence of 5% Pd/C (15 mg) at 10 bar for 18 h with magnetic stirring. A mixture of the syn/anti-5-amino-9-isopropyl-benzonorbornenes is obtained. The identity of the reaction product, its diastereoisomeric purity (in % syn) and yield (in %) are ascertained by means of gas chromatography.

| **Temperature** | **% syn** | **Yield** |
|---|---|---|
| Ambient temperature | 83 | 98 |
| 50°C | 64 | 99 |
| 80°C | 53 | 94 |

### Example P5: Yield in the preparation of syn-enriched 5-amino-9-isopropyl-benzonorbornene using hydrogen chloride as additive:

In an autoclave, 500 mg (2.2 mmol) of isopropylidene-5-nitro-benzonorbornadiene in 5 ml of solvent are exhaustively hydrogenated in the presence of 5% Pd/C (25 mg) at ambient temperature and a pressure of 20 bar for 18 h with magnetic stirring. A mixture of the *syn*/*anti*-5-amino-9-isopropyl-benzonorbornenes is obtained. The identity of the reaction product, its diastereoisomeric purity (in % syn) and yield (in %) are ascertained by means of gas chromatography.

| **Solvent/Additive** | **% syn** | **Yield** |
|---|---|---|
| Methanol, 0.15 equivalent of hydrogen chloride | 79 | 92 |
| Methanol, 0.75 equivalent of hydrogen chloride | 81 | 90 |

Hydrogen chloride is added in the form of a 12% solution in methanol. The amount of hydrogen chloride is expressed in equivalents in relation to isopropylidene-5-nitro-benzonorbornadiene.

### Example P6: Yield in the preparation of syn-enriched 5-amino-9-isopropyl-benzonorbornene using triethylamine as additive:

In an autoclave, 500 mg (2.2 mmol) of isopropylidene-5-nitro-benzonorbomadiene in 5 ml of solvent are exhaustively hydrogenated in the presence of 5% Pd/C (15 mg) at ambient temperature and a pressure of 10 bar for 18 h with magnetic stirring. A mixture of the syn/anti-5-amino-9-isopropyl-benzonorbornenes is obtained. The identity of the reaction product, its diastereoisomeric purity (in % syn) and yield (in %) are ascertained by means of gas chromatography.

| **Solvent/Additive** | **% syn** | **Yield** |
|---|---|---|
| Methanol | 83 | 98 |
| Methanol, 1.6 equivalents of triethylamine | 87 | 98 |

The amount of triethylamine is expressed in equivalents in relation to isopropylidene-5-nitro-benzonorbornadiene.

The starting materials for the process of the present invention are distinguished by ready availability and good handling properties and are moreover reasonably priced.

## Claims

1. A process for the preparation of syn-enriched 5-amino-9-isopropyl-benzonorbornene in which the ratio of compounds of formula la (syn) 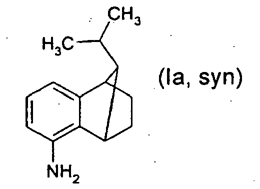 to compounds of formula Ib (anti) 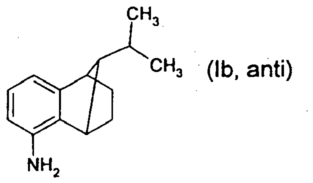 is from 70:30 to 99:1, which comprises reacting a compound of formula II 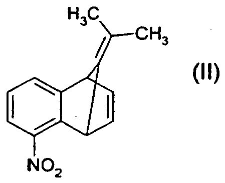 in a pressure vessel with hydrogen in the presence of a palladium catalyst and a solvent, at a pressure of at least 2 bar and a temperature of from 0°C to 45°C, to form syn-enriched 5-amino-9-isopropyl-benzonorbornene.

2. A process according to claim 1, which is carried out at a pressure of from 2 to 50 bar.

3. A process according to claim 1, which is carried out at a pressure of from 7 to 50 bar.

4. A process according to claim 1, which is carried out at a temperature of from 20°C to 45°C.

5. A process according to claim 1, wherein the reaction is carried out in the presence of from 0.01 to 10 equivalents of an additive.

6. A process according to claim 5, wherein the additive is a base.

7. A process according to claim 5, wherein the additive is an organic nitrogen base.

8. A process according to claim 5, wherein the additive is a trialkylamine base.

9. A process according to claim 5, which is carried out at a pressure of from 2 to 50 bar.

## Patentansprüche

1. Verfahren zur Herstellung von syn-angereichertem 5-Amino-9-isopropyl-benzonorbornen, worin das Verhältnis von Verbindungen der Formel Ia (syn) 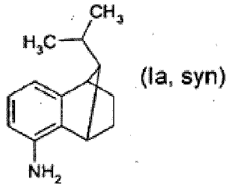 zu Verbindungen der Formel Ib (anti) 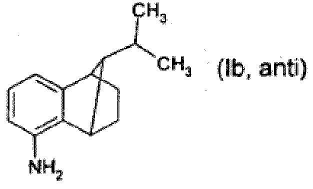 70:30 bis 99:1 beträgt, bei dem man eine Verbindung der Formel II 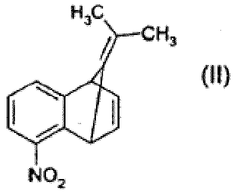 in einem Druckgefäß in Gegenwart eines Palladiumkatalysators und eines Lösungsmittels bei einem Druck von mindestens 2 bar und einer Temperatur von 0°C bis 45°C mit Wasserstoff zu syn-angereichertem 5-Amino-9-isopropyl-benzonorbornen umsetzt.

2. Verfahren nach Anspruch 1, das bei einem Druck von 2 bis 50 bar durchgeführt wird.

3. Verfahren nach Anspruch 1, das bei einem Druck von 7 bis 50 bar durchgeführt wird.

4. Verfahren nach Anspruch 1, das bei einer Temperatur von 20°C bis 45°C durchgeführt wird.

5. Verfahren nach Anspruch 1, bei dem die Umsetzung in Gegenwart von 0,01 bis 10 Äquivalenten eines Additivs durchgeführt wird.

6. Verfahren nach Anspruch 5, bei dem das Additiv eine Base ist.

7. Verfahren nach Anspruch 5, bei dem das Additiv eine organische Stickstoffbase ist.

8. Verfahren nach Anspruch 5, bei dem das Additiv eine Trialkylaminbase ist.

9. Verfahren nach Anspruch 5, das bei einem Druck von 2 bis 50 bar durchgeführt wird.

## Revendications

1. Procédé de préparation de 5-amino-9-isopropyl-benzonorbonène enrichi en syn, dans lequel le rapport entre les composés de formule Ia (syn) 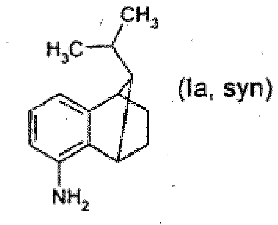 et les composés de formule Ib (anti) 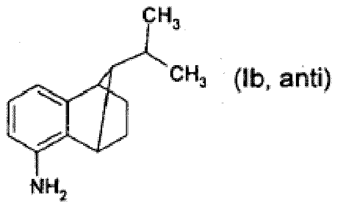 est de 70:30 à 99:1, qui comprend la réaction d'un composé de formule II 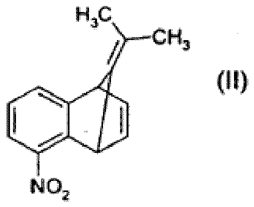 dans un récipient sous pression, avec l'hydrogène en présence d'un catalyseur au palladium et d'un solvant, à une pression d'au moins 2 bar et à une température de 0°C à 45°C, pour former le 5-amino-9-isopropyl-benzonorbornène enrichi en syn.

2. Procédé selon la revendication 1, **caractérisé en ce qu'**il est effectué à une pression de 2 à 50 bar.

3. Procédé selon la revendication 1, **caractérisé en ce qu'**il est effectué à une pression de 7 à 50 bar.

4. Procédé selon la revendication 1, **caractérisé en ce qu'**il est effectué à une température de 20°C à 45°C.

5. Procédé selon la revendication 1, **caractérisé en ce que** la réaction est effectuée en présence de 0,01 à 10 équivalents d'un additif.

6. Procédé selon la revendication 5, **caractérisé en ce que** l'additif est une base.

7. Procédé selon la revendication 5, **caractérisé en ce que** l'additif est une base azotée organique.

8. Procédé selon la revendication 5, **caractérisé en ce que** l'additif est une base trialkylamine.

9. Procédé selon la revendication 5, **caractérisé en ce qu'**il est effectué à une pression de 2 à 50 bar.
